# EUROPEAN PATENT SPECIFICATION

(11) **EP 0 614 465 B1**
(45) Date of publication and mention of the grant of the patent: **17.03.1999**
(21) Application number: 92923486.2
(22) Date of filing: 11.11.1992
(51) Int. Cl.: C07K 14/445, C12N 15/62, A61K 39/015, C12N 7/04

(54) **HYBRID PROTEIN BETWEEN CS FROM PLASMODIUM AND HBsAG**
HYBRIDES PROTEIN ZWISCHEN CS AUS PLASMODIUM UND HBsAG
PROTEINE HYBRIDE ENTRE LA PROTEINE CS DE PLASMODIUM ET L'ANTIGENE DE SURFACE DU VIRUS DE L'HEPATITE B

(30) Priority: 16.11.1991 GB 9124390; 27.02.1992 US 842694
(43) Date of publication of application: 14.09.1994
(73) Proprietor: SMITHKLINE BEECHAM BIOLOGICALS S.A., 1330 Rixensart (BE)
(72) Inventor: DE WILDE, Michel, 89, rue de l'Institut B-1330 Rixensart (BE); COHEN, Joseph SmithKline Beecham Biologicals s.a., B-1330 Rixensart (BE)
(74) Representative: Dalton, Marcus Jonathan William
(86) International application number: EP9202591
(87) International publication number: WO9310152

(56) References cited:
- EP-A- 0 175 261
- EP-A- 0 278 940
- EP-A- 0 343 460
- WO-A-88/10300
- WO-A-90/00402
- US-A- 4 722 840
- US-A- 4 886 782
- AMERICAN JOURNAL OF TROPICAL MEDICINE AND HYGIENE vol. 47, no. 4 SU, 1992, USA pages 96 - 97 WHITE, K. ET AL. 'Induction of cytolytic T cells by recombinantly co-expressed circumsporozoite protein fragment of Plasmodium falciparum and Hepatitis B particles' abstract no 19

## Description

The present invention relates to a novel hybrid protein, its use in medicine, particularly in the prevention of malaria infections and vaccines containing it.

Malaria, is one of the world's major health problems with 2 to 4 million people dying from the disease each year. One of the most acute forms of the disease is caused by the protozoan parasite, Plasmodium falciparum which is responsible for most of the mortality attributable to Malaria.

The life cycle of P. falciparum is complex, requiring two hosts, man and mosquito for completion. The infection of man is initiated by the inoculation of sporozoites in the saliva of an infected mosquito. The sporozoites migrate to the liver and there infect hepatocytes where they differentiate, via the exoerythrocytic intracellular stage, into the merozoite stage which infects red blood cells (RBC) to initiate cyclical replication in the asexual blood stage. The cycle is completed by the differentiation of a number of merozoites in the RBC into sexual stage gametocytes which are ingested by the mosquito, where they develop through a series of stages in the midgut to produce sporozoites which migrate to the salivary gland.

The sporozoite stage of P. falciparum has been identified as a potential target of a malaria vaccine. The major surface protein of the sporozoite is known as circumsporozoite protein (CS Protein). This protein from strain 7G8 has been cloned, expressed and sequenced (Dame et al Science 225 (1984) p593). The protein from strain 7G8 is characterised by having a central immunodominant repeat region comprising a tetrapeptide Asn-Ala-Asn-Pro repeated 37 times but interspersed with four minor repeats Asn-Val-Asp-Pro. In other strains the number of major and minor repeats vary as well as their relative position. This central portion is flanked by an N and C terminal portion composed of non-repetitive amino acid sequences designated as the repeatless portion of the CS protein.

It has been shown that irradiated sporozoites can provide significant protection against experimental human malaria (Am. J. Trop. Med. Hyg. 24: 297-402, 1975). However, production difficulties makes the use of irradiated sporozoite impractical from the point of view of producing a vaccine.

Several groups have proposed subunit vaccines based on the circumsporozoite protein. Two of these vaccines have undergone clinical testing; one is a synthetic peptide, the other is a recombinant protein (Ballou et al Lancet: i 1277 (1987) and Herrington et al Nature 328:257 (1987).

These vaccines were successful in stimulating an anti-sporozoite response. Nonetheless, the magnitude of the response was disappointing, with some vaccinees not making a response at all. Furthermore, the absence of "boosting" of antibody levels on subsequent injections and results of in vitro lymphocyte proliferation assays suggested that T-cells of most of these volunteers did not recognise the immuno-dominant repeat. Nonetheless, one vaccinee in each study did not develop parasitemia.

The present invention provides a new, improved antigen for use in malaria vaccines which not only produces a humoral response, but also a cellular immune response. Preferably the antigen induces the production of neutralising antibodies against the immunodominant repeat. Most preferably, the antigen should also elicit effector T cell mediated immune responses of the CD4⁺ and CD8⁺ cytotoxic T lymphocyte (CTL) type and of the delayed type hypersensitivity type and also, preferably be able to induce T helper (TH) memory cells.

Accordingly, the present invention provides a hybrid protein consisting of substantially all the C-terminal portion of the CS protein, four or more tandom repeats of the immunodominant region, and the Surface antigen from Hepatitis B virus (HBsAg). Preferably the hybrid protein comprises a sequence which contains at least 160 amino acids which is substantially homologous to the C-terminal portion of the CS protein. The CS protein may be devoid of the last 12 amino-acids from the C terminal.

In particular there is provided a protein which consists of a portion of the CS protein of P. falciparum substantially as corresponding to amino acids 210-398 of P. falciparum 7G8 fused in frame via a linear linker to the N-terminal of HBsAg. The linker may comprise a portion of preS2 from HBsAg.

The present invention also provides DNA sequences encoding the proteins of the present invention.

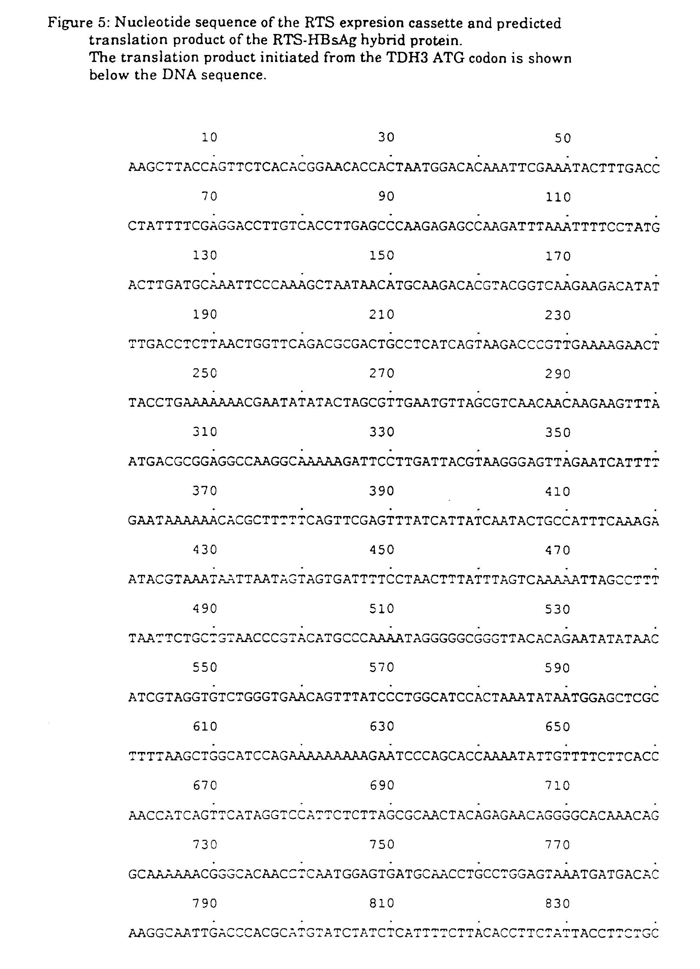

A particularly preferred embodiment is the hybrid protein designated RTS. The amino acid sequence of RTS is shown in figure 5. This hybrid consists of:
° A methionine-residue, encoded by nucleotides 1059 to 1061, derived from the Sacchromyes cerevisiae TDH3 gene sequence. (Musti A.M. et al Gene 1983 25 133-143.
° Three amino acids, Met Ala Pro, derived from a nucleotide sequence (1062 to 1070) created by the cloning procedure used to construct the hybrid gene.
° A stretch of 189 amino acids, encoded by nucleotides 1071 to 1637 representing amino acids 210 to 398 of the circumsporozoite protein (CSP) of Plasmodium falciparum strain 7G8 (Dame et al supra).
° An amino acid (Arg) encoded by nucleotides 1638 to 1640, created by the cloning procedure used to construct the hybrid gene.
° Four amino acids, Pro Val Thr Asn, encoded by nucleotides 1641 to 1652, and representing the four carboxy terminal residues of the hepatitis B virus (adw serotype) preS2 protein (9).
° A stretch of 226 amino acids, encoded by nucleotides 1653 to 2330, and specifying the S protein of hepatitis B virus (adw serotype).

In an alternative embodiment there is provided a hybrid protein designated RTS* which was generated using the CSP gene sequence from P. falciparum NF54 (Mol. Biochem Parisitol. 35 : 185-190, 1989) and comprises substantially all of the region 207 to 395 of the CS protein from P falciparum NF54.

In particular RTS* consists of :
- A Methionine, encoded by nucleotides 1059 to 1061, derived from the TDH3 gene sequence.
- Three amino acids, Met Ala Pro, derived from a nucleotide sequence (1062 to 1070) created by the cloning procedure used to construct the hybrid gene.
- A stretch of 189 amino acids, encoded by nucleotides 1071 to 1637 representing amino acids 207 to 395 of the circumsporozoite protein (CSP) of Plasmodium falciparum strain NF54 (Mol.Biochem.Parasitol, 35:185-190, 1989).
- An amino acid (Gly) encoded by nucleotides 1638 to 1640, created by the cloning procedure used to construct the hybrid gene.
- Four amino acids, Pro Val Thr Asn, encoded by nucleotides 1641 to 1652, and representing the four carboxy terminal residues of the hepatitis B virus (adw serotype) preS2 protein (Nature 280:815-819, 1979).
- A stretch of 226 amino acids, encoded by nucleotides 1653 to 2330, and specifying the S protein of hepatitis B virus (adw serotype) (Nature 280:815-819, 1979).

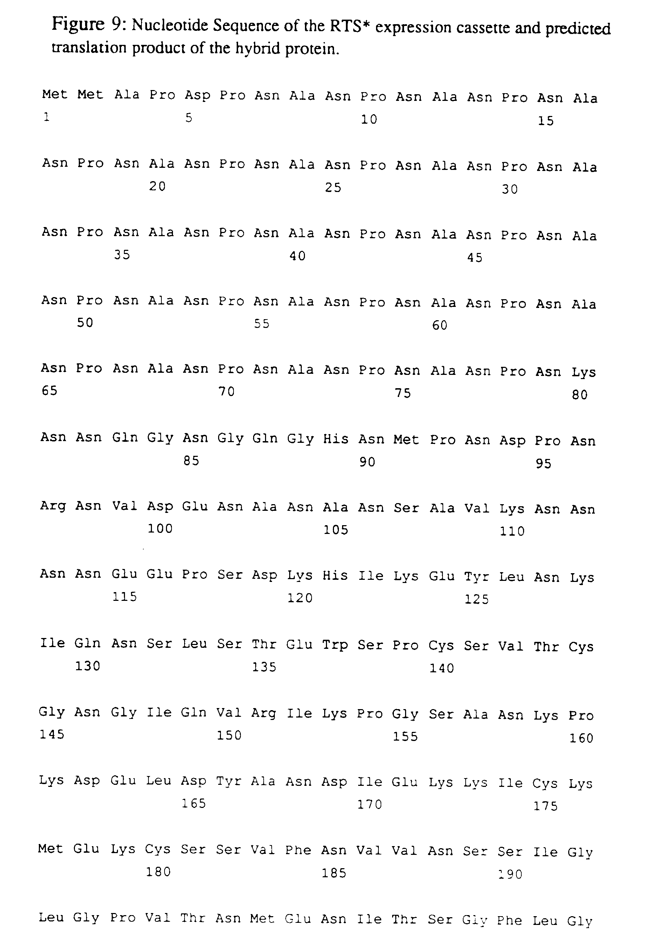

The amino acid sequence of RTS* is depicted in figure 9.

An expression cassette containing RTS* was constructed and comprises the following features:
- A promoter sequence, extending from nucleotide 1 through 1058, derived from the S.cerevisiae TDH3 gene.
- An open reading frame starting at nucleotide 1059 and extending to nucleotide 2330. This open reading frame is immediately followed by a translational stop codon, TAA (nucleotides 2331 to 2333). The open reading frame encodes the amino acids specifying the hybrid RTS* protein.
- A transcription termination sequence contained within the sequence extending from base pair 2334 to 3504, derived from the S. cerevisiae ARG3 gene.

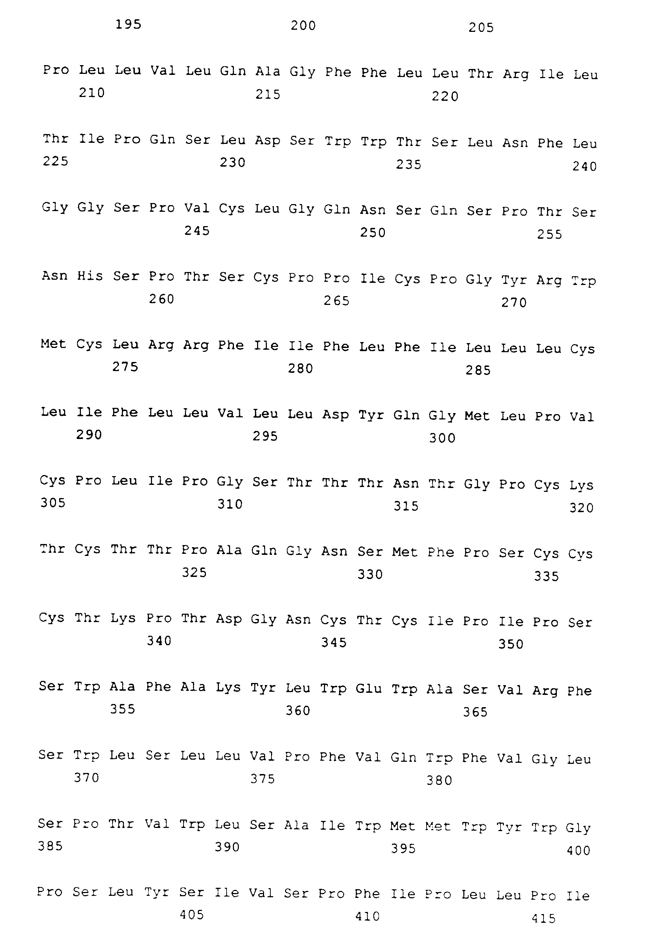

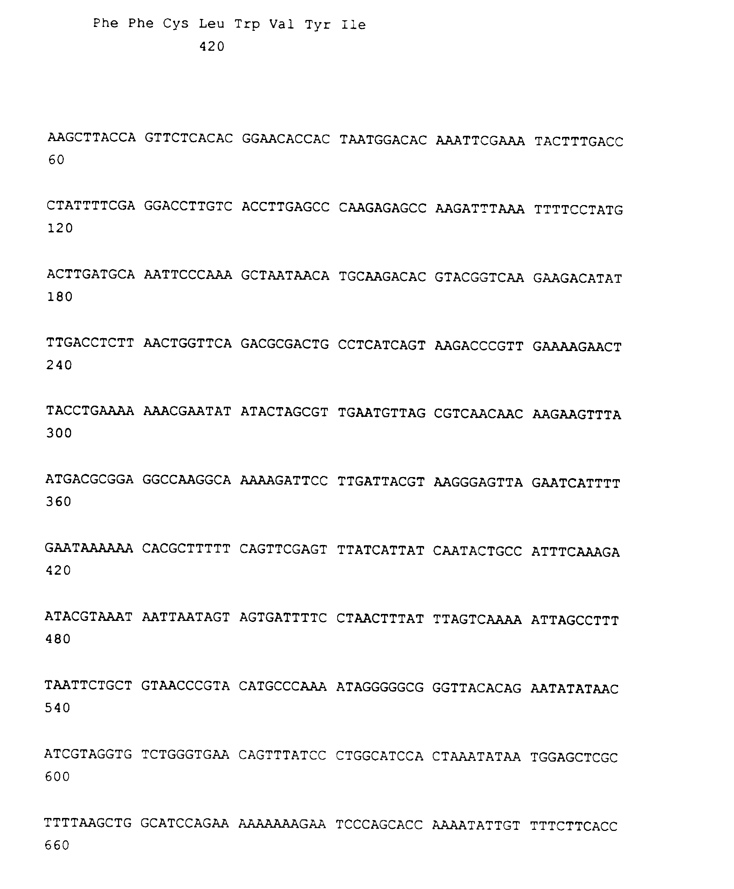

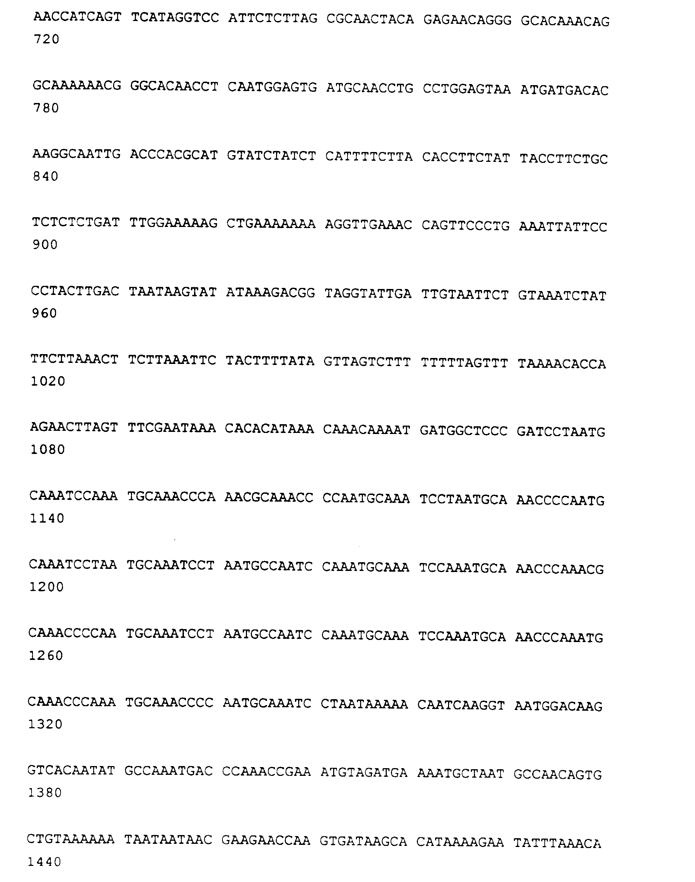

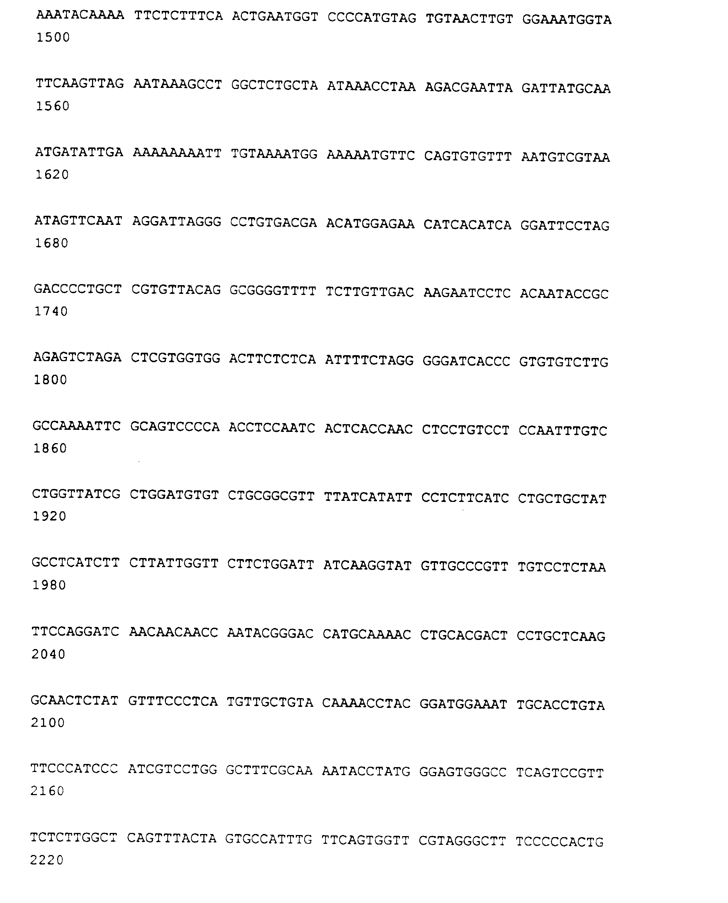

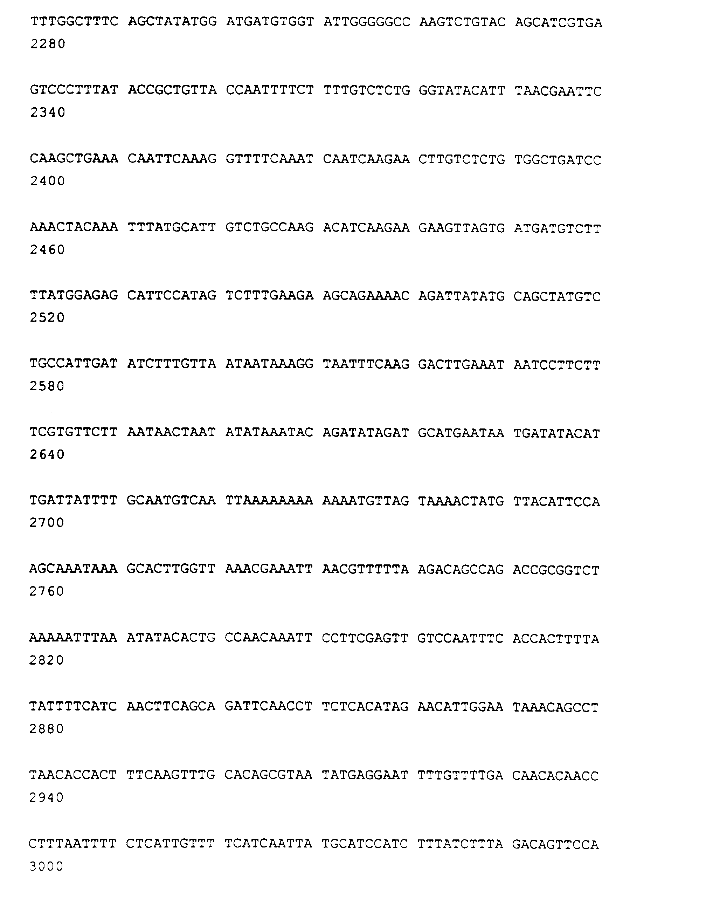

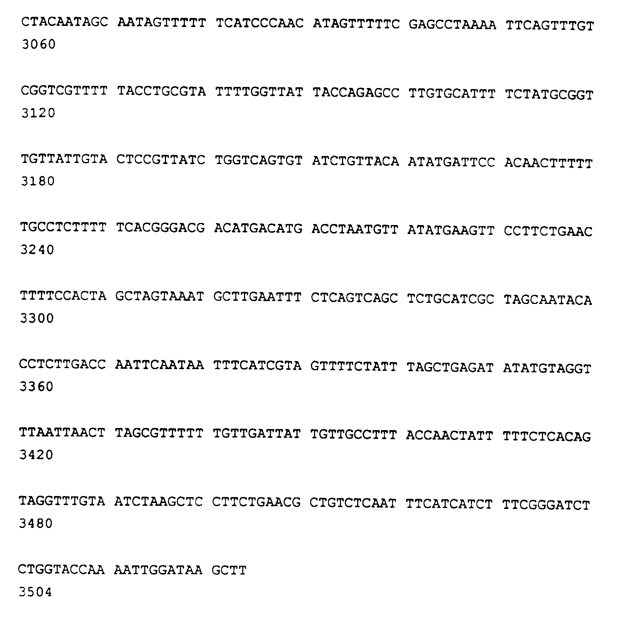

The nucleotide sequence is depicted in figure 10.

The DNA sequences encoding the proteins of the present invention are, in a preferred embodiment flanked by transcriptional control elements, preferably derived from yeast genes and incorporated into an expression vector.

Such vectors are a further aspect of the invention. A preferred promoter is the promoter from the S. cerevisiae TDH3 gene Musti et al surpra).

The invention also relates to a host cell transformed with a vector according to the invention. Host cells can be prokaryotic or eukaryotic but preferably, are yeast, such as S. cerevisiae. In such a host, the hybrid protein, for example RTS will be expressed as lipoprotein particle. The chosen recipient yeast strain preferably already carries in its genome several integrated copies of an hepatitis B S expression cassette. The resulting strain syntheses two polypeptides, S and RTS (or other hybrid protein of the invention), that spontaneously co-assemble into mixed (for example RTS, S or RTS*, S) lipoprotein particles. These particles, advantageously present the CSP sequences of the hybrid at their surface. These mixed particles also form part of the present invention. Advantageously the ratio or RTS: S or RTS* : S in these mixed particles is 1:4.

The present invention also relates to vaccines comprising an immunoprotective amount of a protein or particle according to the invention in admixture with a suitable diluent or carrier.

In the vaccine of the invention, an aqueous solution of the hybrid may be used directly. Alternatively, the protein with or without prior lyophilisation can be mixed or absorbed with any of the known adjuvants which include but are not limited to alum, muramyl dipeptide, saponins such as Quil A.

An immunostimulant may alternatively or in addition be included. In a preferred embodiment this immunostimulant will be 3 Deacylated monophosphoryl lipid A (3D-MPL).

3 Deacylated monophosphoryl lipid A is known from US patent No. 4,912,094 and UK patent application No. 2,220,211 (Ribi) and is available from Ribi Immunochem, Montana, USA.

The protein of the present invention may also be encapsulated into microparticles such as liposomes.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877.

Conventional adjuvants may be used, but a preferred immunostimulant is 3-deacylated monophosphoryl lipid A (3D-MPL).

Typically when 3D-MPL is used the antigen and 3D-MPL are delivered with alum or presented in an oil in water emulsion or multiple oil in water emulsions. The incorporation of 3D-MPL is advantageous since it is a stimulator of effector T-cells responses.

Accordingly a preferred embodiment of the present invention provides a vaccine comprising a hybrid protein as herein described, preferably RTS, or RTS* in combination with 3D-MPL and a carrier. Typically the carrier will be an oil in water emulsion or alum.

In a most preferred embodiment the hybrid protein is presented as a particle or mixed particle as herein described.

The amount of the protein of the present invention present in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each does will comprise 1-1000µg of protein, preferably 1-200 µg most preferably 10-100µg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists. The immune response to the protein of this invention is enhanced by the use of adjuvant and or an immuno stimulant.

The proteins of the present invention are preferably expressed in yeast, and especially those belonging to the genus Saccharomyces.

A further aspect of the present invention is to provide a process for the preparation of hybrid protein of the invention, which process comprises expressing DNA sequence encoding the protein, in a suitable host, preferably a yeast, and recovering the product.

It is particularly preferred to express the protein of the invention in a Sacchromyces strain. When RTS, for example is expressed in such strains it spontaneously assembles into multimeric lipoprotein particles.

These particles are highly immunogenic and induce a strong humoral response, as well as immunological memory and also are capable of inducing effector T cells of the CTL and DTH types.

A further aspect of the invention lies in a method of treating a patient susceptible to plasmodium infections by administering an effective amount of a vaccine as hereinbefore described.

### Example 1

### 1. CONSTRUCTION OF THE RTS.S strain RIT4383

The S. cerevisiae strain, RIT4383 used for production of particles containing both the S and RTS polypeptides, carries separate expression cassettes for each protein. The S gene expression cassette has been integrated in 5 to 6 copies at least two sites in genome using a linear integration vector with homology to resident Ty retrotransponsons. The RTS gene expression cassette has been integrated in 2 to 3 copies at one or 2 sites in the genome, using a linear integration vector similar to the one employed for the integration of the S gene cassette. Expression from both types of cassette is driven by a promoter derived from the yeast TDH3 gene.

### 1.1 CONSTRUCTION OF THE S EXPRESSION CASSETTE AND INTEGRATIVE VECTOR (pRIT13034)

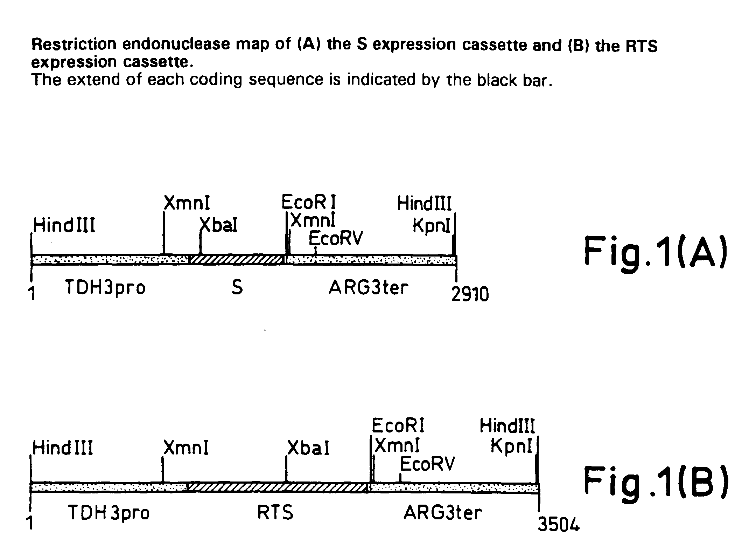

The S gene expression cassette (Figure 1A) identical to that found in strain RIT4376 (1) and consists of a 1058 bp TDH3 promoter fragment, 681 bp of S gene sequence, a 6 bp spacer containing an EcoR1 linker and a 1165 bp fragment carrying the Arg3 transcription terminator. The S coding sequence was derived by subcloning from a complete genomic clone (pRIT10616) of a virus of (adw serotype).

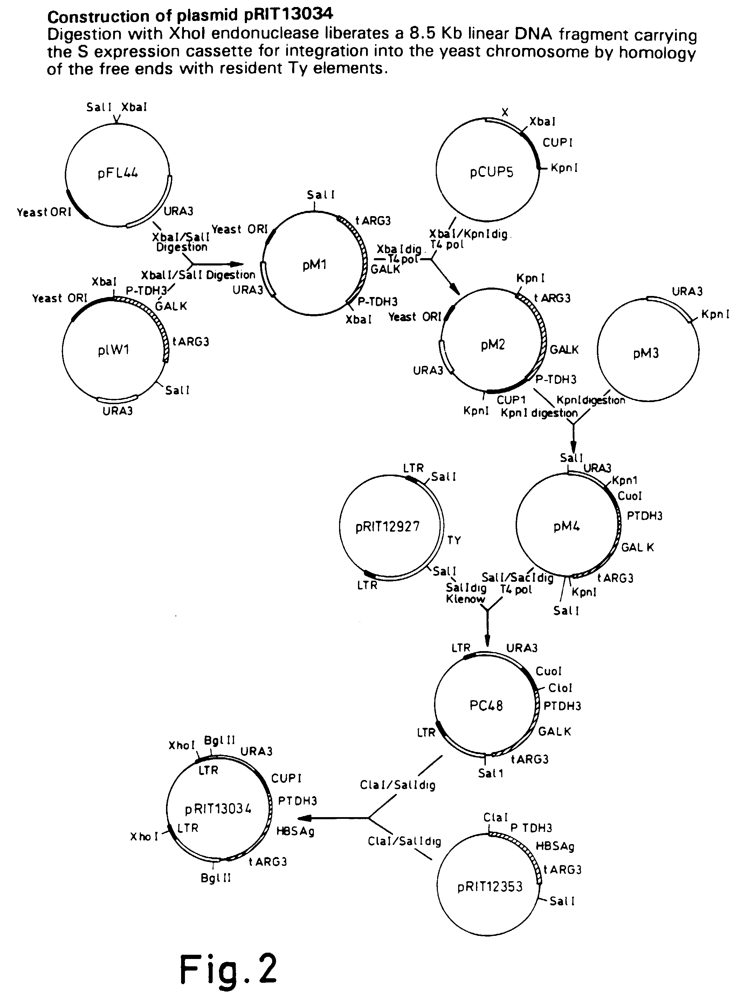

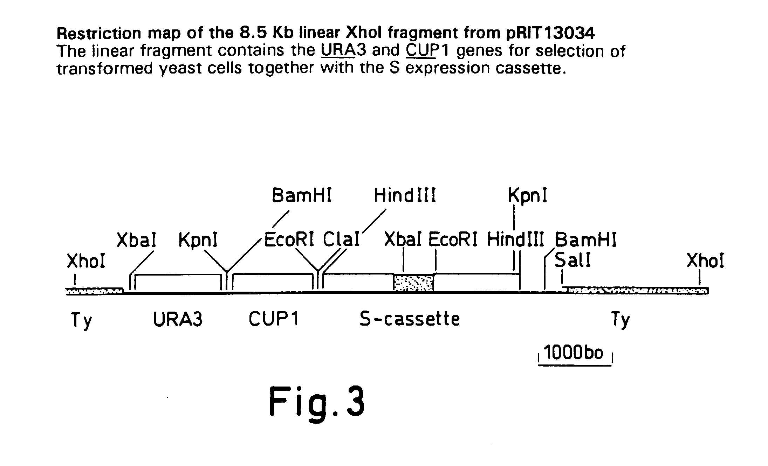

The structure of the Ty vector, pRIT13034, used for integration of the S expression cassette into the yeast genome, is shown in Figure 2. The construction and use of this type of vector for integration of expression cassettes in the yeast genome is described in Jacobs et al. (2). pRIT13034 contains the S gene cassette, the URA3 gene and the CUP1 gene inserted within a copy of a Ty element cloned on pUC9. The URA3 gene and the CUP1 gene both provide selectable markers that allow to distinguish transformed yeast colonies from untransformed colonies. pUC9 is described by Vieira & Messing (3), the URA3 fragment is from pFL44 (F. Lacroute, CNRS, Strasbourg), the CUP1 fragment is from pCUP5 (T. Butt, SKF Labs, Philadelphia), the Ty element is from Juniaux et al. (4) and the S gene cassette from pRIT12353. Digestion of pRIT13034 with XhoI endonuclease liberates the 8500bp linear fragment shown in Figure 3 which can integrate into the genome by homologous recombination of the free ends with resident Ty elements.

### Example 2

### 2. CONTRUCTION OF STRAIN Y1295

The recipient strain, EJ cup1D-3d (trp1, leu2, ura3, cup1, gal1D, MATα) was used for initial introduction by transformation of the linear vector fragment from pRIT13034. This strain contains a single disrupted cup1 locus, cup1.

After transformation with the linear XhoI fragment, Ura⁺ colonies were isolated and screened for copper resistance. The more resistant transformants had integrated two to five copies of the vector as determined by Southern blotting analysis. Two transformant colonies copies were retained, MS9 with an estimated 3 to 4 copies and MS23 with 4 to 5 copies of the integrated linear vector. Strain MS23 was then crossed with strain EJ cup1D-7b (trp1, ura3, cup1, gal1D, MATα) and a haploid ascospore recovered to give strain MS23-2a.

This strain was then backcrossed to MS9 and a Leu⁻, Trp⁻ haploid segregant obtained (MS54-2c) containing 5 to 6 copies of the integrated expression cassette. Southern blotting showed that MS54-2C contained 4 to 5 tandem copies of the integration vector at one locus and a further single copy integrated at a different locus. All 5 to 6 copies of the expression cassette were intact as digestion of total yeast cell DNA with HindIII endonuclease to liberate the cassette fragment and Southern blotting with an S gene specific probe gave a single 3 kb band as expected. A spontaneous Trp⁺ revertant of this strain was obtained, MS54-2c-T, and given the laboratory accesssion number Y1295.

### Example 3 CONSTRUCTION OF THE RTS-EXPRESSION CASSETTE

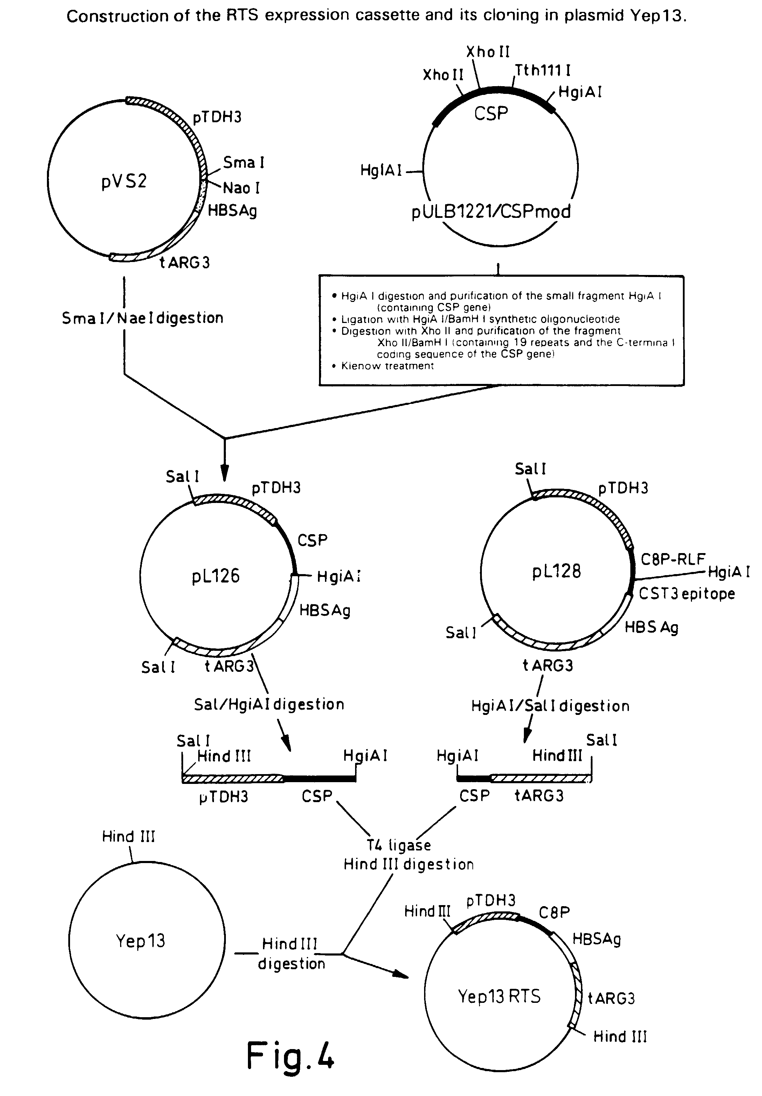

The expression cassette for the RTS hybrid protein was constructed by a multistep cloning procedure and was cloned in the E.coli yieast shuttle vector Yep13(6) yielding a plasmid Yep13RTS (Fig.4). The structure of the cassette is shown in figure 1B. Its entire nucleotide sequence was determined either by direct sequencing (as for the coding sequence and parts of the flanking control sequences) or by consultation of the relevant literature (as for parts of the promotor and terminator sequences). The DNA sequence is illustrated in figure 5. It contains the following elements:
A promotor sequence, extending from nucleotide 1 through 1058, derived from the S. cerevisiae TDH3 gene.
An open reading frame starting at nucleotide 1059 and extending to nucleotide 2330. This open reading frame is immediately followed by a translational stop codon, TAA (nucleotides 2331 to 2333). The open reading frame encodes the amino acids specifying the hydrid RTS protein.
A transcription termination sequence contained within the sequence extending from base pair 2334 to 3504, derived from the S. cerevisiae ARG3 gene (Crabeel et al EMBO J. 1983 2: 205-212).

The amino acid sequence of the hydrid RTS protein, encoded by nucleotides 1059 to 2330 is indicated in figure 5 and contains the following elements:
A methionine-residue, encoded by nucleotides 1059 to 1061, derived from the TDH3 gene sequence.
Three amino acids, Met Ala Pro, derived from a nucleotide sequence (1062 to 1070) created by the cloning procedure used to construct the hybrid gene.
A stretch of 189 amino acids, encoded by nucleotides 1071 to 1637 representing amino acids 210 to 398 of the circumsporozoite protein (CSP) of Plasmodium falciparum strain 7G8 (8).
An amino acid (Arg) encoded by nucleotides 1638 to 1640, created by the cloning procedure used to construct the hybrid gene.
Four amino acids, Pro Val Thr Asn, encoded by nucleotides 1641 to 1652, and representing the four carboxy terminal residues of the hepatitis B virus (adw serotype) preS2 protein (9).
A stretch of 226 amino acids, encoded by nucleotides 1653 to 2330, and specifying the S protein of hepatitis B virus (adw serotype).

### Example 4 CONSTRUCTION OF THE RTS CASSETTE INTEGRATIVE VECTOR pRIT13539

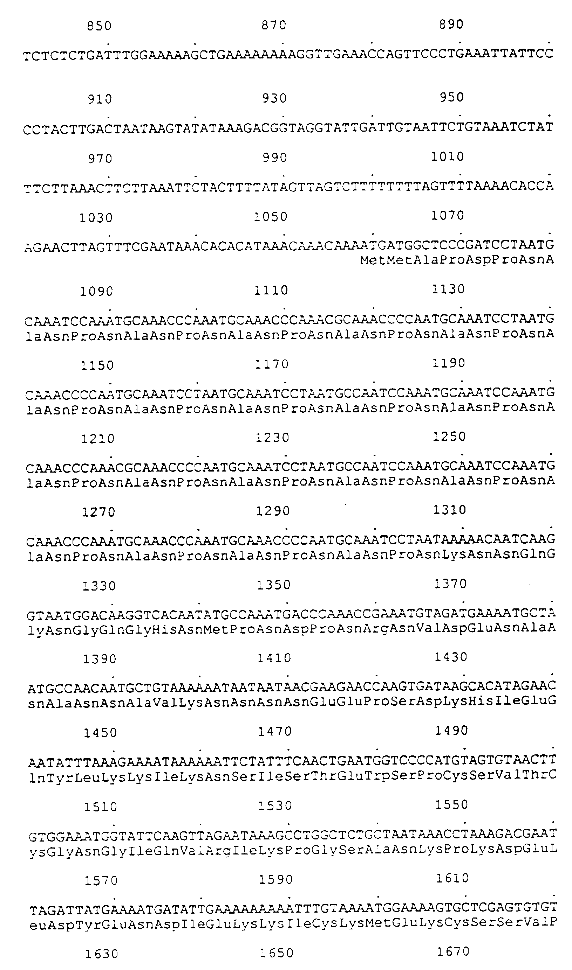

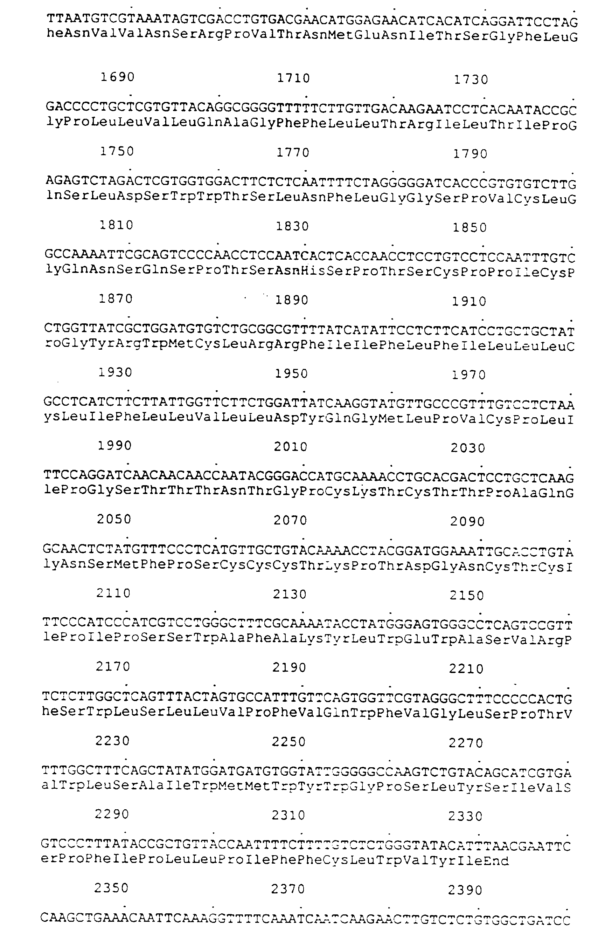

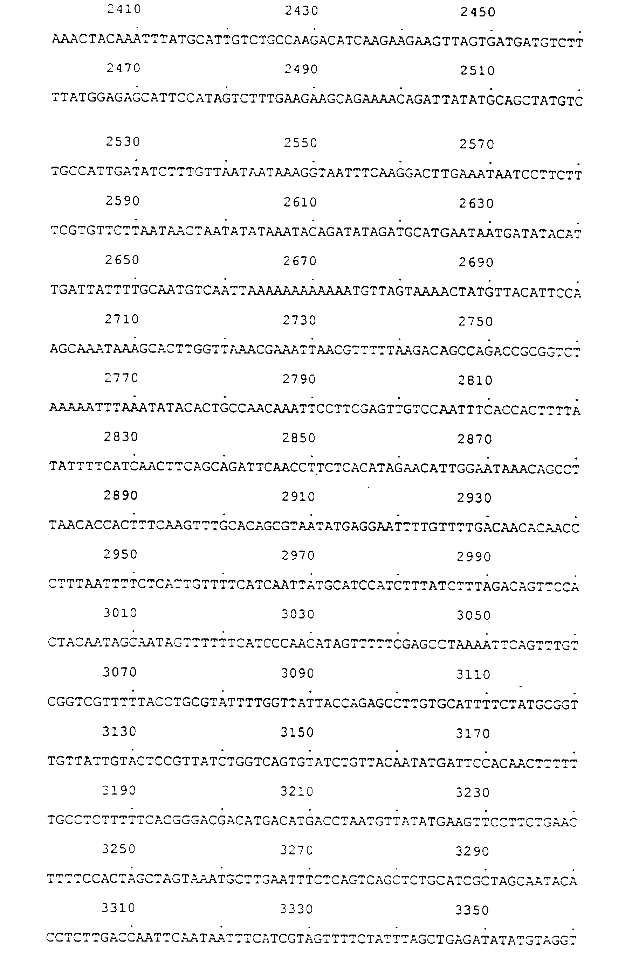

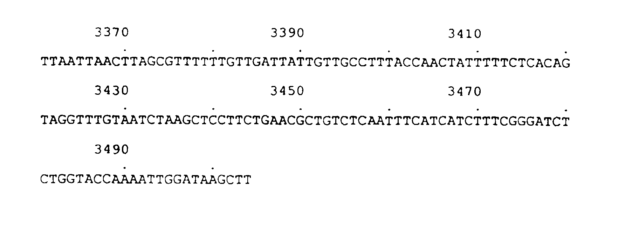

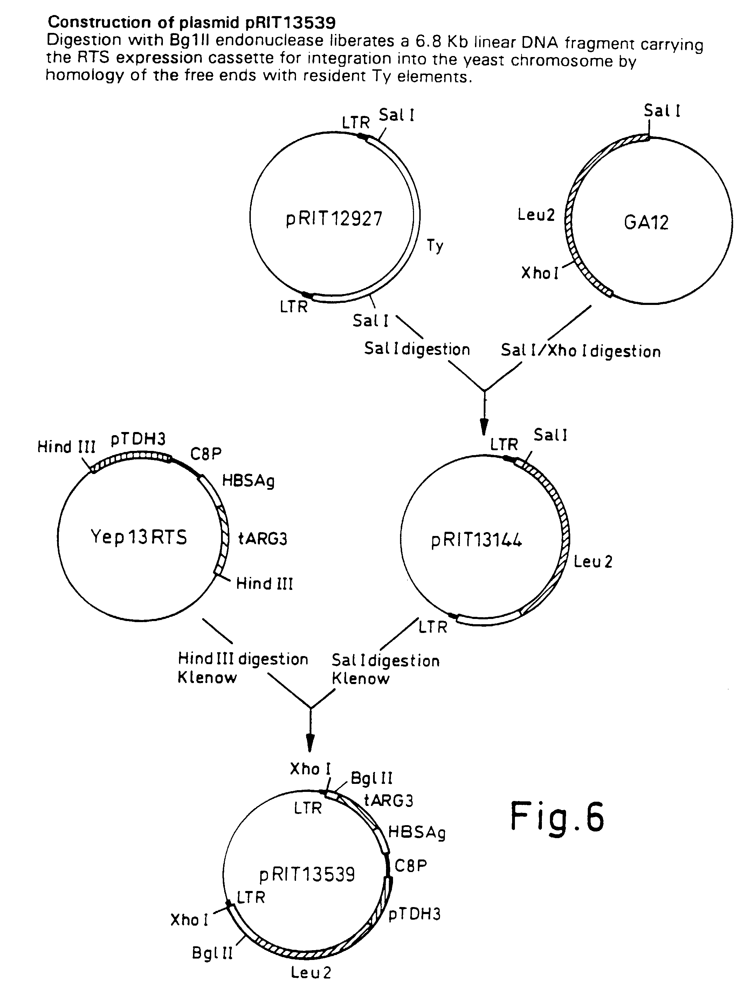

The Construction of the RTS Cassette integrative vector pRIT13539 is shown on figure 6.

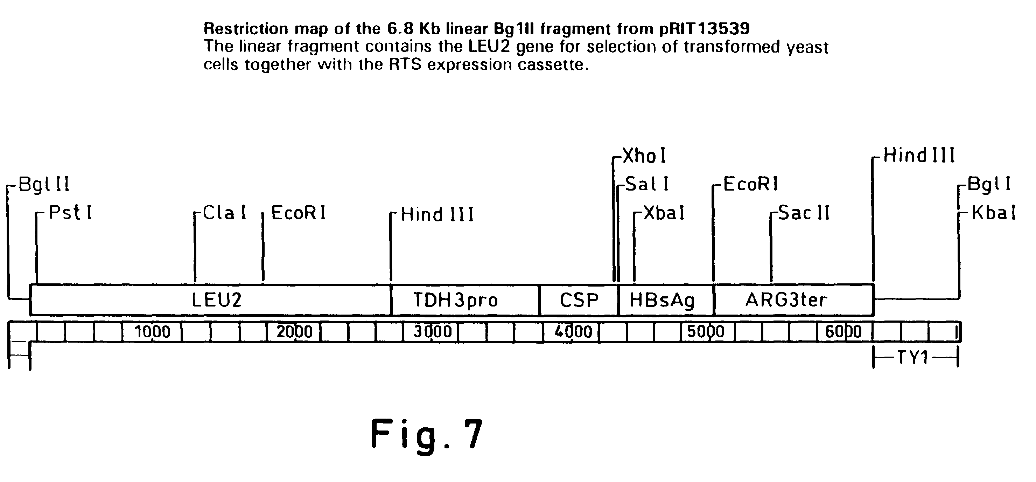

The RTS expression cassette was inserted on the Ty based integrative vector pRIT13144. This vector is a derivative of pRIT12927 (2) in which the LEU2 gene was inserted in the SalI site of the Ty element as a SalIXhoI fragment isolated from the vector pCV9 (7). Thus, after insertion of the RTS expression cassette into pRIT13144, the resulting plasmid, pRIT13539, contains, in addition to the expression cassette, the yeast LEU2 gene as selective marker (figure 5). Digestion of pRIT13539 with Bg1II endonuclease liberates a 6.800 bp linear fragment shown in figure 7 which can integrate into the genome by homologous recombination of the free ends with resident Ty elements.

### Example 5 TRANSFORMATION OF STRAIN Y1295 AND GENERATION OF STRAIN RIT4383 (Y1530)

To obtain a strain expressing both S and RTS proteins, Y1295 was transformed with the 6800 bp linear Bg1II fragment (figure 7) with selection for Leu⁺ colonies. Several integrants containing sets of both expression cassettes present in the genome at various ratio were obtained. One selected transformant, expressing the RTS and S protein in a ratio of approximately 1:4 was given the official designation RIT4383 (the laboratory accession number is Y1530).

### Example 5b Transformation of Strain Y1295 and Generation of Strain Y1631.

A similar (to RTS) construct was generated using the CSP gene sequence derived from P.falciparum strain NF54 (Mol.Biochem.Parasitol.35:185-190, 1989). The fusion protein obtained will be designated RTS∗ to distinguish it from the construct obtained with the CSP derived from P.falciparum strain 7G8.

The sequence of the expression cassette is shown in figure 9. It contains the following elements:
- A promoter sequence, extending from nucleotide 1 through 1058, derived from the S.cerevisiae TDH3 gene.
- An open reading frame starting at nucleotide 1059 and extending to nucleotide 2330. This open reading frame is immediately followed by a translatiional stop codon, TAA (nucleotides 2331 to 2333). The open reading frame encodes the amino acids specifying the hybrid RTS* protein.
- A transcription termination sequence contained within the sequence extending from base pair 2334 to 3504, derived from the S. cerevisiae ARG3 gene.

This expression cassette encoding this RTS* fusion protein was transformed and integrated into the genome of yeast strain Y1295, using the same approach as described previously for the RTS construct. Transformed clones expressing both the S and RTS* proteins were obtained. One clone was selected expressing the two proteins in a ratio of approximately 4S:1RTS∗. The clone was given the laboratory accession number Y1631.

### Example 6

### PRELIMINARY CHARACTERIZATION OF THE STRAIN RIT4383

### 6.1 Analysis by immunoblotting

Cell free extracts prepared from RIT 4383 were analysed by immunoblot using various antibodies:
- a monoclonal antibody directed toward the S protein (Mab HBS1)
- a monoclonal antibody directed toward the repeat part of the RTS protein (Mab 167)
- a rabbit serum directed toward the repeat-less sequences of the RTS protein (rabbit serum no. 20).

In yeast strain RIT4383, two expressed products were recognized by monoclonal antibody HBS1: a 24KD protein corresponding to the S protein and a 46KD RTS hybrid protein. The RTS hybrid protein is also detected by antibodies directed toward repeat and non-repeat epitopes of the CSP. These results indicate clearly that strain RIT4383 simultaneously expresses the two S and RTS antigens at a ratio RTS/S of approximatively 1:4.

### 6.2 CsC1 density gradient centrifugation

The formation of particles in strain RIT4383 was analyzed by CsC1 density gradient centrifugation. Crude extract (± 15 mg of total protein) was analyzed on a 10 ml 1.5 M CsC1 gradient (68 hours at 40.000 rpm, + 8°C in a Beckman 50 Ti rotor). Fractions (0.5ml) were collected and analyzed by a radioimmunoassay specific for HBsAg (AUSRIA), by an RTS specific ELISA and by immunoblot using an anti-HBsAg monoclonal antibody.

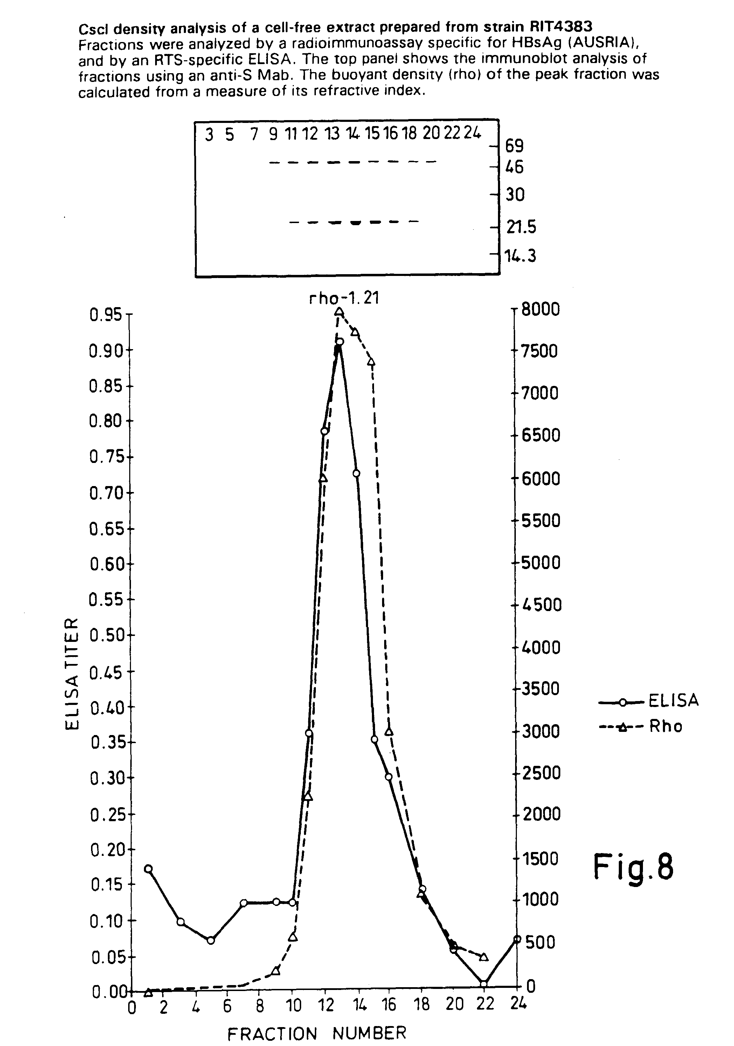

As shown in Figure 8, ELISA, RIA and Western blot peaks appear at the same fraction (no. 13) of the gradient corresponding to a buoyant density of rho = 1.21 suggesting that mixed particles, containing both S and RTS monomers, are formed in this strain.

### Example 7

### PREPARATION OF THE SEED LOTS

### Production procedure for master seed lot

Strain Y1530 (RIT4383) is first grown for 48 hours at 30°C in Petri dishes containing 20 ml sterile YNB (Difco) supplemented with dextrose (0.1%) and 1.8% (w/v) agar (Difco). The surface growth is suspended into sterile YNB broth (Difco) supplemented with dextrose (1%) and glycerol (10%). This suspension is distributed under aspectic conditions into 2 ml sterile polypropylene stoppered tubes (1 ml per tube) and stored at -70°C.

### Production procedure for working seed lot

One master seed tube is rapidly thawed, and its contents are streaked with a platinum loop on Petri dishes prepared as described above. After incubation at 30°C for 63 hours, part of the surface growth is transferred to a 2 L conical flask containing 400 ml of sterile YNB broth (Difco) supplemented with dextrose (2%). The culture is incubated at 30°C for 24 hours before being centrifuged (15 min at 6300 x g) under aseptic conditions. The pellet is resuspended into sterile YNB broth (Difco) supplemented with dextrose (1%) and glycerol (10%). This is distributed under aspectic conditions into 2 ml sterile glass stoppered tubes (0.5 ml per tube) and stored at -70°C.

### Example 8: FERMENTATION

### Preparation of the inoculum

### (a) Growth on solid medium

One vial of the working seed lot is rapidly thawed and spread onto Petri dishes containing sterile YNB broth (Difco) supplemented with dextrose (1%) and 1.8% (w/v) agar (Difco). The Petri dishes are incubated for 48 hours at 300°C.

### (b) Growth of inoculum

The surface growth of one Petri dish is suspended in sterile YNB broth (Difco) supplemented with dextrose (2%) and distributed equally into two conical flasks (2 L,400 ml liquid per flask). The flasks are incubated for 24 hours at 30°C on a rotary shaker.

### Fermentation

The fermentor vessel (20 L total volume), containing 5 L of dieionized water supplemented with (NH₄)₂SO₄ (40 g) is sterilized in-situ at 121°C for 30 minutes using clean, pre-filtered steam at 2 bar g pressure. After cooling to room temperature, the liquid volume in the fermentor is adjusted to 4L, and 1 L of filter-sterilized HB4 solution is added. The fermentation is begun by adding the inoculum (800 ml) from the two conical flasks.

The fermentation is run using the fed-batch technique whereby the culture is continuously fed with a solution of the following composition.
- 5 L HB4 solution;
- 4 L dextrose 80% (sterilized at 121°C).

The culture density increases by aerobic growth at 30°C and pH 5 (maintained by addition of NH₄OH). Dissolved oxygen is maintained about 50% saturation by adjustment of airflow and agitation speed. The rate of addition of the feed is predetermined to maximize growth rate and minimize formation of by-product ethanol.

The fermentation is stopped after 40-90 hours. At the end of the fermentation, the total culture volume is 10-18 L, and the dry cell weight is between 30 and 100 g/L. The culture is rapidly cooled down to 15-25°C, and the yeast cells are recovered from the broth by centrifugation. The concentrated yeast cells are washed once with phosphate buffer (50 mM) before being re-centifuged and subsequently stored at -70°C in polyethylene bags.

| **HB4 Medium Composition** | |
|---|---|
| Component | Quality |
| KH₂PO₄ | 41.00 g |
| MgSO₄.7H₂O | 23.50 g |
| CaCl₂.2H₂O | 4.70 g |
| NaCl | 0.30 g |
| FeCl₃.6H₂O | 50.00 mg |
| H₃BO₃ | 28.00 mg |
| MnSO₄.H₂O | 22.40 mg |
| ZnSO₄.7H₂O | 22.40 mg |
| Na₂MoO₄.2H₂O | 11.20 mg |
| KI | 5.60 mg |
| CaCl₂.6H₂O | 5.10 mg |
| CuSO₄.5H₂O | 2.24 mg |
| Biotin | 2.70 mg |
| Folic acid | 2.70 mg |
| Inositol | 2.70 mg |
| Ca Pantothenate | 0.54 mg |
| Pyridoxin.HC1 | 0.54 mg |
| Thiamin.HC1 | 0.54 mg |
| Niacin | 1.20 mg |
| P-amino benzoic acid | 0.60 mg |
| Riboflavin | 0.60 mg |
| HC1 (37°C) | 5.00 ml |
| Deionized water (up to) | 1.00 litre |

### Example 9: Extraction and Purification of RTS/S

### EXTRACTION PROCEDURE

### 9.1 Preparation of cell suspension

Frozen concentrated yeast cells (at -70°C) are thawed down to - 30°C overnight and subsequently thawed out to 5-15°C by placing the polyethylene bags containing the cells in water (10-20°C). A yeast cell suspension is made with a phosphate buffer solution (pH 8.0) containing the following ingredients : Ethylenediamine tetraacetic acid 9EDTA), pMethylsulfonyl Fluoride (PMSF), isopropanol and tween 20.

### 9.2 Cell disruption

Cells are disrupted in a bead mill containing lead-free glass beads (0.49-0.70 mm diameter). The grinding chamber is cooled by circulation of refrigerating fluid at -20°C, in such a way that the temperature of the homogenate at the outlet of the grinding chamber does not exceed 15°C. The liquid flowrate is 6 L/hour and the agitation speed is 3000 rpm. This process is performed twice. The pH of the resulting homogenate is 6.7 to 7.5.

### 9.3 Polyethyleneglycol clarification

Polyethyleneglycol 400 (PEG 400) is added to the disrupted cell suspension to give a final concentration of 10% (30 minutes below 10°C, pH 6.1) and a partially-clarified supernatant is recovered by centrifugation (J21B Beckman centrifuge, JA10 rotor at 17,000 g for 1 hour).

### 9.4 Methanol clarification

Methanol is added at pH 7 to the PEG-clarified antigen to give the proportion of 1 volume of methanol for 5 volumes of PEG-clarified antigen. The clarified antigen is obtained at 17,000 g for 20 minutes by cenrifugation (J21B Beckman centrifuge, JA10 rotor).

### 9.5 Adsorption/Desorption on colloidal silica.

The crude antigen is adsorbed overnight onto 1.5% (w/v) colloidal silica (Aerosil 380, Degussa) at 4°C.

After washing (3 times) the pellet by successive centrifugation and resuspension in NaCl 0.9% (w/v), the antigen is desorbed using a 10mM pyrophosphate buffer, pH 9.5, containing 1% TWEEN 20.

The desorbing buffer volume corresponds to 1/8 of the methanol clarified antigen solution. The desorbed antigen solution is recovered by ultracentrifugation in a L 8.70 Beckman ultracentrifuge rotor R19 at 50,000 g for 60 minutes.

### 9.6 Diafiltration

Before the purification steps, the desorbed antigen is washed by ultrafiltration with 5 volumes of urea 6M, NaCl 500 mM, TRIS-HC1 20 mM at pH 8.5 in order to eliminate much of the proteic and lipidic contaminants.

Then the buffer is exchanged in the same system (Ultrasette, FILTRON fitted with polysulfone membranes with a 300 kD nominal cut-off) with 5 volumes of TRIS-HC1 10 mM (ph 8.1).

### 9.7 Ion exchange chromatography on DEAE-TSK 650 (M)

The clarified solution is applied to an anion-exchange column (DEAE-TSK 650 (M)) equilibrated in a 10 mM TRIS-buffer, pH 8.1. After washing successively with 2 volumes of 10 mM TRIS-HC1 buffer pH 8.1 and 3 volumes of 10 mM TRIS-HC1 buffer pH 8.1 supplemented with 40 mM NaCl, the antigen is desorbed with less than one volume of 10 mM TRIS-HC1 buffer pH 8.1 containing 150 mM NaCl. The antigen-containing fractions are pooled.

### 9.8 Hydrophobic interaction chromatography on Butyl-TSK 650 (M)

After NaCl addition up to a final concentration of 650 mM Nacl, the antigen solution is loaded on a Butyl-TSK 650 (M) column equilibrated with a 20 mM TRIS-HC1 buffer, 600 mM Nacl (pH 8.1). Most of the antigen passes through while most of the impurities bind to the gel.

### 9.9 Concentration by ultrafiltration

The HIC pool is concentrated by ultrafiltration in an Ultrasette system (FILTRON) fitted with polysulfone membranes with a 300 kDanominal cut-off.

### 9.10 Ultracentrifugation in a CsCl gradient

CsCl is added to the Butyl-TSK pool to give a final concenration of 1.5 M.

After 65 hours in a 50.2 Ti Beckman rotor at 270,000 g, the antigen-containing fractions are collected.

### 9.11 Size exclusion chromatography on SEPHACRYL S300 (HR Type)

In order to exchange the buffer and to eliminate low molecular weight contaminants, the antigen solution is applied to a SEPHACRYL S300 HR column. The elution buffer is 10 mM phosphate, containing 150 mM NaCl (pH 7.4).

### STERILE FILTRATION

After dilution to between 150 and 400 µg Lowry/ml and pH adjustment to 6.8, the purified antigen is sterilized by filtration through a 0.22 µm sterile filter. The resulting solution contains purified RTS/S particles.

### Example 10: IMMUNOLOGICAL CHARACTERIZATION OF RTS/S

### 10.1 Antigenicity

In order to test the antigenicity of the RTS/S particles, a number of ELISA's were performed, combining monoclonal antibodies directed against the different epitopes.

The monoclonal antibodies (MoAbs) used are :
- MoAb R10 :
   - specific for the repeat sequence (NANP) of the CSP region.
   - IgM isotype.
- MoAb RL117:
   - specific for the non-repeat sequence of the CSP region.
- MoAb RF1:
   - specific for the S sequence of the HBsAg.
   - IgG1 isotype.

MoAbs R10 and RL117 were prepared in house by fusion of Sp2/OAg 14 myeloma cells with splenocytes of Balb/C mice immunized with partially pure RTS-like particles containing both repeat and non-repeat regions of the CSP sequence.

Three batches of the candidate RTS/S vaccine were analyzed : batches Nos. 24M31, 24M32 and 24M34.

### 10.2 Reaction with monoclonal antibody R10 (anti-repeat)

The anti-repeat MoAb R10 was used in a "sandwich" ELISA. Samples to be tested were incubated in microtiter plates previously coated with MoAb R10. The same MoAb coupled to peroxidase was then added. After one hour incubation at 37°C and washing, color was developed by addition of Orthophenylene-diamine-H202. Absorbance was measured at 490 nm and plotted versus the antigen concentration.

### Results

The three batches : 24M31, 24M32 and 24M34 reacted positively and consistently with MoAb R10, thus confirming the presence and accessibility of the repeat epitopes on the RTS/S particles. The amount of antigen necessary to reach 50% of maximum binding was 51.2, 38.2 and 60.6 ng for batches 24M31, 24M31 and 24M34 respectively.

### 10.3 Reaction with monoclonal antibody RL117 (anti-repeatless)

The reactivity of MoAb RL117 with RTS,S particles was analyzed in a "sandwich" ELISA test. MoAb RL117 was coupled to peroxidase and used for the detection of the RTS/S particles, while MoAb R10, specific for the repeat region, was used for the capture of particles.

Briefly, samples to be tested were incubated in microtiter plates previously coated with MoAb R10. RL117 coupled to peroxidase was then added, and, after one hour incubation at 37°C and washing, the coloration was developed by addition of Orthophenylene-diamine-H202. Absorbance was measured at 490 nm and plotted versus the antigen concentration.

### Results

The three batches : 24M31, 24M32 and 24M34 reacted positively and consistently with MoAbs RL117 and R10, thus confirming the presence and accessibility of repeat and ono-repeat epitopes on the same RTS/S particles. The amount of antigen necessary to reach 50% of maximum binding was 169.2, 117.6 and 181.1 ng for batches 24M31, 24M32 and 24M34 respectively

### 10.4 Reaction with monoclonal antibody RF1 (anti-S)

The presence of S-epitopes in RTS/S particles was shown by a "sandwich" ELISA test, using MoAb RF1 coupled to peroxidase for detection. The R10 MoAb was used directly onto the microtiter plates in order to capture the RTS,S particles.

In summary, samples to be tested were incubated in microtiter plates previously coated with MoAb R10. MoAb RF1 coupled to peroxidase was then added. After incubation at 37°C for 1 hour and washing, color development was allowed by addition of

Orthophenylene-diamine-H₂O₂. Absorbance was measured at 490 nm and plotted versus the antigen concentration.

### Results

The three batches : 24M31, 24M32 and 24M34 reacted positively and consistently with MoAbs RF1 and R10, thus confirming the presence and accessibility of the repeat and Sepitopes on the same RTS/S particles. The amount of antigen necessary to reach 50% of maximum binding was 52.3, 55.2 and 106.2 ng for batches 24M31, 24M32 and 24M34 respectively.

### Example 11: IMMUNOGENICITY IN VIVO OF RTS/S PARTICLES

### 11.1 Immunogenicity studies

Studies on the immunogenicity of (RTS,S) particles were performed in mice and in Cercopithecus aethiop monkeys.

In mice, anti-CSP antibodies were analyzed by ELISA test using the R32tet32 antigen for the detection of the anti-repeat antibodies. R32tet32 consists of 32 copies of the NANP (major) repeat fused to a 32 amino acid portion of the tetracyclin resistance gene of plasmid pBR322. The recombinant antigen is produced in Escherichia coli. Antibodies (Abs) directed against the non repeated sequence of the CS protein were measured by ELISA test sing the RLF antigen. The RLF antigen consists of the complete CS protein flanking region devoid of repeat fused to the first 81 amino acids of the NS1 protein of influenza virus. The RLF antigen is produced in E. coli. Mice sera were serially diluted, starging at 1:100, and titers are expressed as the reciprocal of the dilution corresponding to an optical density of 1.0 in the ELISA test (1). Measurements of anti-CSP Abs were performed on individual sera and the geometric mean titer (GMT) was calculated.

In order to analyze the anti-carrier response, anti-HBs antibody titers were also measured (pooled sera only).

In mice experiments, Balb/C mice (H-2d hanlotype) usually used for the immunogenicity of HBsAg were also used to evaluate the immunogenicity of RTS,S particles. The intraperitoneal (i.p.) and the subcutaneous (s.c.) routes of immunization were compared and the effect of the immunostimulant 3-deacylated monophosphoryl lipid A (3D-MPL) on the immune response was also tested.

The immunogenicity of the (RTS,S) vaccine was also tested in a similar way in Cercopithecus aethiops monkeys.

Selected individual or pooled sera were also tested for their capacity to inhibit the in vitro invasion of a human hepatoma cell line (HepG2) by P. falciparum sporozoites (ISI assay (2)).

### 11.2 Immunogenicity in mice

### EXPERIMENT 1 : Immunogenicity of clinical (RTS,S) batches adsorbed on A1(OH)₃

### Method

### Immunization :

Groups of 10 Balb/C mice were injected twice intraperitoneally at one month interval with 1 µg of each of three (RTS,S) batches previously adsorbed on A1(OH)₃ (batches 24M/31, 24M/32 and 24M/34). Control mice were injected with HBsAg (Engerix-B, batch ENG611B4). On days 30 and 45, the mice were bled and the antibody titers were measured on individual sera.

### Serological methods :

The anti-R32tet32 and the anti-RLF titers were measured by ELISA using respectively R32tet32 and RLF as coating antigens. The microplates were incubated with the serum sample (12 two-fold serial dilutions starting at 1:100) to be tested. Mouse Abs were reacted with biotinylated anti-mouse Ig followed by streptavidin : biotinylated horseradish peroxidase complex and orthophenylenediamine/H₂O₂. The optical density was measured at 490 nm. The titers were expressed as the reciprocal of the dilution corresponding to an optical density of 1.0 (50 % maximal binding). For each group of mice, the geometric mean titer (GMT) was calculated. The anti-HBs antibody titer was calculated according to the Hollinger formula (3) and expressed in mIU/ml.

### Results

### Anti-CSP response :

Strong anti-R32tet32 and anti-RLF responses were observed for each (RTS,S) batch tested. No significant difference between the batches was noted. A remarkable booster effect was observed after the second dose. Mice immunized with HBsAg ("Engerix-B") were used as negative control in this experiment.

### Anti-HBs response :

The (RTS,S) batches induce antibodies directed against the HBsAg carrier protein. The assay was performed on pooled sera only.

### EXPERIMENT 2: Effect of the immunostimulant 3D-MPL on the immunogenicity of (RTS,S) particles in Balb/C mice.

We analyzed the effect of the 3D-MPL on the immunogenicity of the (RTS,S) vaccine in mice. Both the intraperitoneal (i.p.) and the subcutaneous (s.c.) routes of immunization were tested.

### A. Immunization by the ip route

### Method

### Immunization:

Groups of 10 Balb/C mice were injected twice intraperitioneally at one month interval with 1 µg of (RTS,S) (batch 24M/34) adsorbed on A1(OH)₃ alone or on A1(OH)₃ + 3D-MPL (50 µg/dose). Control mice were injected with NaC1 150 mM. One days 30 and 45, the mice were blend and the antibody titers measured on individual sera.

### Serological method:

The serological methods were the same as in the first experiment described above.

### Results

### Anti-CSP response:

The (RTS,S) vaccine batch induces strong anti-R32tet32 and anti-RLF responses in both formualtions. In all cases, a significant booster effect is observed following the second immunization. Titers obtained with the formulation containing 3D-MPL are in all cases higher than the aluminium alone formulation, and a statistically significant increase was observed in the primary anti-R32tet32 response (p = 0.02). A group of mice injected with NaC1 150 mM was used as negative control in this experiment.

### Anti-HBs response:

Both formualtions of the (RTS,S) vaccine, injected by the i.p. route, induced a strong anti-HBs response. A significant booster effect was observed following second immunization with either formulation.

### B. Immunization by the s.c. route

### Method

### Immunization:

Groups of 10 Balb/C mice were injected twice subcutaneously at one month interval with 1 µg of (RTS,S), (batch 24M/34) adsorbed on A1(OH)₃ alone or on A1(OH)₃ + 3D-MPL (50 µg/dose). Control mice were injected with NaC1 150 mM. One days 30 and 45, the mice were blend and the antibody titers measured on individual sera.

The serological methods were the same as in the first experiment described above.

### Results

### Anti-CSP response:

The (RTS,S) vaccine batch induced positive response against R32tet32 and RLF in both formulations. In all cases, a significant booster effect was observed following the second immunization. Statistically significant higher titers were observed with the 3D-MPL formulation on day 45, both for the anti-R32tet32 and anti-RLF responses (p = 0.18 and p = 2.9 respectively). In general, however, titers were lower than those obtained with the i.p. route. A group of mice injected with NaC1 150 mM was used as negative control in this experiment.

### Anti-HBs response:

Both formulations of the (RTS,S) vaccine, injected by the s.c. route induced a good anti-HBs response. A significant booster effect was observed following second immunization with either formulation. As observed for the anti-CSP responses, the anti-HBs response was lower by this route of immunization as compared to the i.p. route.

### 11.3 Immunogenicity in Cercopithecus aethiops

The immunogenicity was evaluated in Cercopithecus aethiops monkeys with clinical batch 24M/32 adsorbed on A1(OH)₃.

### Methods

### Immunization:

Five monkeys wer injected intramuscularly on days 0, 28 and 84 with 20 µg of (RTS,S) particles adsorbed on A1(OH)₃ (0.5 mg A1+++). The animals were bled on days 0, 14, 28, 42, 56, 66 and 98. Antibodies directed against R32tet32, RLF and HBs antigens were measured.

### Serological methods:

A anti-R32tet32 and the anti-RLF antibody titers were measured by ELISA using respectively the R32tet32 and the RLF antigens coated on microplates. The plates were then incubated with the serum sample to be tested (12 two-fold serial dilutions starting at 1:10). Monkey antibodies were detected by biotinylated anti-human Ig followed by streptavidin biotinylated horseradish peroxidase complex and orthophenylenediamine/H₂O₂. The optical density was measured at 490 nm. The titers wer expressed as the reciprocal of the dilution corresponding to an optical density of 1.0 (50% maximal binding). For each group, the geometric mean titer (GMT) was calculated. The anti-HBs antibody titers were calculated according to the Hollinger formula (Hollinger et al., 1982) and expressed in mIU/ml.

### Results

### Anti-CSP response:

The (RTS,S) vaccine induced a positive response against both R32tet32 and RLF antigens in all 5 monkeys. A significant booster effect was observed 14 days following second immunization (day 42). A slow decrease of antibody titers was then observed up to the third immunization. The titers again increase 14 days following the third immunization (day 98), expect in the case of the anti-RLF response of monkey Jo 352.

The anti-R32tet32 titers reached after the third immunization (day 98) are not however higher than those observed after the second immunization (day 42).

In the case of the anti-RLF response (with the exception of monkey JO 353), an increase of titers is observed following the third immunization (day 98) relative to post second immunization levels (day 42).

### Anti-HBs response:

All monkeys raised an anti-HBs response with significant booster effects following second (day 42) as well as third (day 98) immunization.

### Biological activity of antibodies raised against the (RTS.S) particle

As a measure of the biological function of the antibodies induced by the (RTS,S) vaccine, pooled mice and individual monkeys sera were tested by the Inhibition of Sporozoite Invasion (ISI) assay (Hollingdale et al., 1984). This assay measured the capacity of the anti-CSP antibodies to inhibit the in vitro invasion of a human hepatoma cell line (HepG2) by P. falciparum sporozoites.

### Results

The results of this experiment are presented in Tables 1 and 2. The ISI data are expressed as % inhibition relative to the activity of a pre-immune control serum taken as 0% inhibition. For reference, the anti-R32tet32 and RLF antibody titers of the tested sera are included. Table 1 shows that all mice sera tested have very high ISI activity. Table 2 shows that all 5 monkeys have also a very ISI activity on day 98 compared with the corresponding immune serum.

### Conclusion

The (RTS,S) particles induced, both in mice and monkeys, a high antibody response directed against the repeat and non-repeat epitopes of the CSP and against the S protein of the HBsAg carrier.

The primary antibody response in mice was enhanced by the presence of 3D-MPL.

Antibody titers obtained after intraperitoneal injection were higher than those obtained after immunization by the subcutaneous route.

The antibodies elicited in the two animal species effectively prevented invasion of cultured human hepatoma cells by P. falciparum sporozoites.

**Table 1:**

| ISI activity of sera from Balb/C mice immunized with TS.S | | | |
|---|---|---|---|
| Pool of Mice sera | A-R32tet32 Titer | A-RLF Titer | ISI (%) |
| Pool i.p A1+3D-MPL Day 45 | 606544 | 242408 | 100% |
| Pool i.p Alum only Day 45 | 87005 | 160284 | 98% |
| Pool i.p A1+3D-MPL Day 45 | 15333 | 99002 | 94% |
| Pool s.c A1+3D-MPL Day 45 | 5102 | 20453 | 86% |
| Pool of Negative Controls | 205 | 205 | 0% |

**Table 2:**

| ISI activity of individual monkey sera immunized with RTS.S | | | | |
|---|---|---|---|---|
| Monkey # | Serum | A-R32tet32 titer | A-RLF titer | ISI (%) |
| J0 352 | DAY 0 | <50 | <50 | 0% |
| | DAY 98 | 1762 | 1250 | 98% |
| J0 353 | DAY 0 | <50 | <50 | 0% |
| | DAY 98 | 1574 | 32218 | 90% |
| J0 354 | DAY 0 | <50 | <50 | 0% |
| | DAY 98 | 3751 | 31034 | 98% |
| J0 356 | DAY 0 | <50 | <50 | 0% |
| | DAY 98 | 1495 | 18544 | 95% |
| J0 357 | DAY 0 | <50 | <50 | 0% |
| | DAY 98 | 1420 | 30727 | 98% |

### Reference

(1) Harford N, Cabezon T, Colau B, et al., "Construction and Characterization of a Saccharomyces Cerevisiae Strain (RIT4376) Expressing Hepatitis B Surface Antigen", Postgrad Med J 63, Supp. 2: 65-70, 1987.
(2) Jacobs E, Rutgers T, Voet P, et al., "Simultaneous Synthesis and Assembly of Various Hepatitis B Surface Proteins in Saccharomyces cerevisiae", Gene 80: 279-291, 1989.
(3) Vieira J and Messing J, "The pUC plasmids, an M13mp7-Derived System for Insertion Mutagenesis and Sequencing with Synthetic Universal Primers", *_Gene 19: 259-268, 1982.
(4) Juniaux Embo J 1: 1125-1131, 1982.
(5) Hinnen A, Hicks JB, and Fink GR, "Transformation of Yeast", Proc Natl Acad Sci USA 75: 1929-1933, 1980.
(6) Broach JR, Strathern JN, and Hicks JB, "Transformation in Yeast Development of a Hybrid Cloning Vector and Isolation of the CAN 1 Gene", Gene 8: 121-133, 1979.
(7) Zhang H, et al., "Double Stranded SDNA Sequencing as a Choice for DNA Sequencing", Nucleic Acids Research 16: 1220, 1988.
(8) Dame JB, Williams JL. Mc Cutchan TF, et al., "Structure of the Gene Encoding the Immunodominant Surface Antigen on the Sporozoites of the Human Malaria Parasite Plasmodium falciparum", Science 225: 593-599, 1984.
(9) Valenzuela P, Gray P, Quiroga M, et al., "Nucleotide Sequences of the Gene Coding for the Major Protein of Hepatitis B Virus Surface Antigen", Nature 280: 815-819, 1979.

## Claims

1. A hybrid protein consisting of all or substantially all the C-terminal portion of the CS protein of Plasmodium, four or more tandem repeats of the CS protein immunodominant region, and the surface antigen from Hepatitis B Virus (HBsAg).

2. A hybrid protein consisting of a sequence of CS protein of P.falciparum substantially as corresponding to amino acids 210-398 of P.falciparum 7G8 CS protein or 207-395 of P. falciparum NF54 CS protein fused in frame via a linear linker to the N-terminal of HBsAg.

3. A hybrid protein as claimed in claim 1 wherein the CS protein is fused to N-terminal of HBsAg.

4. A hybrid protein consisting of the following amino acid sequences:
a) an N-terminal methionine residue
b) Met Ala Pro
c) a stretch of 189 amino acids corresponding to amino acids 210 to 398 of CS protein P.falciparum 7G8
d) Arg
e) Pro Val Thr Asn from hepatitis B Pre S₂ protein
f) a stretch of 226 amino acids specifying the S protein of hepatitis B virus

5. A hybrid protein consisting of the following amino-acid sequences:
a) an N-terminal methionine
b) Met Ala Pro
c) a stretch of 189 amino acids corresponding to amino acids 207 to 395 of CS protein P.falciparum NF54
d) Gly
e) Pro Val Thr Asn from hepatitis B PreS₂ protein
f) a stretch of 226 amino acids specifying the S protein of hepatitis B virus.

6. A DNA sequence encoding a hybrid protein as claimed in any one of claims 1 to 5..

7. A vector containing a DNA sequence as claimed in claim 6 said sequence being linked to transcriptional control elements.

8. A host transformed with a vector of claim 7.

9. A host as claimed in claim 8 wherein the host is S. cerevisiae.

10. A host as claimed in claim 9 additionally transformed with a gene encoding the Hepatitis B surface antigen.

11. A multimeric lipoprotein particle comprising a hybrid protein of any one of claims 1 to 5.

12. A mixed multimeric lipoprotein particle comprising a hybrid protein of any one of claims 1 to 5 and Hepatitis B surface antigen.

13. A particle as claimed in claim 12 wherein the ratio of hybrid protein to surface antigen is approximately 1:4.

14. A vaccine comprising an immunoprotective amount of a particle or protein according to any of claims 1 to 5 or claims 11 to 12 in admixture with a suitable diluent or carrier.

15. A vaccine as claimed in claim 14 additionally comprising a saponin adjuvant.

16. A vaccine as claimed in claim 14 additionally comprising 3-Deacylated monophosphoryl lipid A.

17. A vaccine as claimed in claim 16 additionally comprising alum.

18. A vaccine as claimed in claim 16 presented in an oil in water emulsion.

19. A vaccine according to any one of claims 14 to 18, for use in the treatment of a patient susceptible to plasmodium infections.

20. Use of a vaccine according to any one of claims 14 to 18 in the manufacture of a medicament for use in the treatment of a patient susceptible to plamodium infections.

21. A process for the production of a hybrid protein according to any of claims 1 to 5 which process comprises expressing a DNA sequence encoding the protein in a suitable host and recovering the product.

22. A process for the production of particles as claimed in any one of claims 11 to 13 comprising expressing a DNA sequence encoding a protein of claims 1 to 4 and hepatitis B surface antigen in a Sacchromyces strain.

## Patentansprüche

1. Hybridprotein, das aus dem vollständigen oder im wesentlichen vollständigen C-terminalen Teil des CS-Proteins von Plasmodium, vier oder mehr Wiederholungen in Tandemanordnung des immundominanten Bereichs des CS-Proteins und dem Oberflächenantigen des Hepatitis B-Virus (HBsAg) besteht.

2. Hybridprotein, das aus einer Sequenz des CS-Proteins von P. falciparum besteht, die im wesentlichen den Aminosäuren 210 bis 398 des CS-Proteins von P. falciparum 7G8 oder den Aminosäuren 207 bis 395 des CS-Proteins von P. falciparum NF54 entspricht und im Leserahmen über einen linearen Linker mit dem N-Terminus von HBsAg verknüpft ist.

3. Hybridprotein nach Anspruch 1, wobei das CS-Protein mit dem N-Terminus von HBsAg verknüpft ist.

4. Hybridprotein, das aus den folgenden Aminosäuresequenzen besteht:
a) einem N-terminalen Methioninrest
b) Met Ala Pro
c) einem Abschnitt von 189 Aminosäuren, die den Aminosäuren 210 bis 398 des CS-Proteins von P. falciparum 7G8 entsprechen
d) Arg
e) Pro Val Thr Asn des Hepatitis B-Prä-S₂-Proteins
f) einem Abschnitt von 226 Aminosäuren, der das S-Protein des Hepatitis B-Virus spezifiziert.

5. Hybridprotein, das aus den folgenden Aminosäuresequenzen besteht:
a) einem N-terminalen Methionin
b) Met Ala Pro
c) einem Abschnitt von 189 Aminosäuren, die den Aminosäuren 207 bis 395 des CS-Proteins von P. falciparum NF54 entsprechen
d) Gly
e) Pro Val Thr Asn des Hepatitis B-Prä-S₂-Proteins
f) einem Abschnitt von 226 Aminosäuren, der das S-Protein des Hepatitis B-Virus spezifiziert.

6. DNA-Sequenz, die ein Hybridprotein nach einem der Ansprüche 1 bis 5 codiert.

7. Vektor, der eine DNA-Sequenz nach Anspruch 6 enthält, wobei die Sequenz an transkriptionelle Kontrollelemente gebunden ist.

8. Wirt, der mit einem Vektor nach Anspruch 7 transformiert ist.

9. Wirt nach Anspruch 8, wobei der Wirt S. cerevisiae ist.

10. Wirt nach Anspruch 9, der zusätzlich mit einem das Hepatitis B-Oberflächenantigen codierenden Gen transformiert ist.

11. Multimeres Lipoproteinpartikel, das ein Hybridprotein nach einem der Ansprüche 1 bis 5 umfaßt.

12. Gemischtes multimeres Lipoproteinpartikel, das ein Hybridprotein nach einem der Ansprüche 1 bis 5 und das Hepatitis B-Oberflächenantigen umfaßt.

13. Partikel nach Anspruch 12, wobei das Verhältnis von Hybridprotein zu Oberflächenantigen ungefähr 1:4 ist.

14. Impfstoff, der eine immunprotektive Menge eines Partikels oder Proteins nach einem der Ansprüche 1 bis 5 oder 11 bis 12 im Gemisch mit einem geeigneten Verdünnungsmittel oder Träger umfaßt.

15. Impfstoff nach Anspruch 14, der zusätzlich ein Saponin-adjuvans umfaßt.

16. Impfstoff nach Anspruch 14, der zusätzlich 3-desacyliertes Monophosphoryllipid A umfaßt.

17. Impfstoff nach Anspruch 16, der zusätzlich ein Alaun umfaßt.

18. Impfstoff nach Anspruch 16, der in einer Öl-in-Wasser-Emulsion dargeboten wird.

19. Impfstoff nach einem der Ansprüche 14 bis 18 zur Verwendung bei der Behandlung eines für Plasmodiuminfektionen anfälligen Patienten.

20. Verwendung eines Impfstoffs nach einem der Ansprüche 14 bis 18 zur Herstellung eines Arzneimittels zur Verwendung bei der Behandlung eines für Plasmodiuminfektionen anfälligen Patienten.

21. Verfahren zur Herstellung eines Hybridproteins nach einem der Ansprüche 1 bis 5, das die Expression einer das Protein codierenden DNA-Sequenz in einem geeigneten Wirt und die Gewinnung des Produkts umfaßt.

22. Verfahren zur Herstellung von Partikeln nach einem der Ansprüche 11 bis 13, das die Expression einer das Protein nach einem der Ansprüche 1 bis 4 und das Hepatitis B-Oberflächenantigen codierenden DNA-Sequenz in einem Saccharomyces-Stamm umfaßt.

## Revendications

1. Protéine hybride consistant en la totalité ou pratiquement la totalité de la partie C-terminale de la protéine CS de Plasmodium, quatre unités répétées en tandem ou plus de la région immunodominante de la protéine CS, et l'antigène de surface provenant du virus de l'hépatite B (HBsAg).

2. Protéine hybride consistant en une séquence de protéine CS de *P. falciparum* pratiquement telle que celle qui correspond aux acides aminés 210 à 398 de la protéine CS de *P. falciparum* 7G8, ou 207 à 395 de la protéine CS de *P. falciparum* NF54, fusionnée en un cadre par l'intermédiaire d'un linker linéaire au N-terminal de HBsAg.

3. Protéine hybride suivant la revendication 1, dans laquelle la protéine CS est fusionnée au N-terminal de HBsAg.

4. Protéine hybride consistant en les séquences d'acides aminés suivantes :
a) un résidu méthionine N-terminal,
b) Met Ala Pro
c) une longueur de 189 acides aminés correspondant aux acides aminés 210 à 398 de la protéine CS de *P. falciparum* 7G8,
d) Arg
e) Pro Val Thr Asn provenant de la protéine Pré S₂ de l'hépatite B,
f) une longueur de 226 acides aminés spécifiant la protéine S du virus de l'hépatite B.

5. Protéine hybride consistant en les séquences d'acides aminés suivantes :
a) une méthionine N-terminale,
b) Met Ala Pro,
c) une longueur de 189 acides aminés correspondant aux acides aminés 207 à 395 de la protéine CS de *P. falciparum* NF54,
d) Gly,
e) Pro Val Thr Asn provenant de la protéine Pré S2 de l'hépatite B,
f) une longueur de 226 acides aminés spécifiant la protéine S du virus de l'hépatite B.

6. Séquence d'ADN codant une protéine hybride suivant l'une quelconque des revendications 1 à 5.

7. Vecteur contenant une séquence d'ADN suivant la revendication 6, ladite séquence étant liée à des éléments de contrôle transcriptionnels.

8. Hôte transformé avec un vecteur suivant la revendication 7.

9. Hôte suivant la revendication 8, dans lequel l'hôte est *S. cerevisiae.*

10. Hôte suivant la revendication 9, de plus transformé avec un gène codant l'antigène de surface de l'hépatite B.

11. Particule de lipoprotéine multimérique comprenant une protéine hybride suivant l'une quelconque des revendications 1 à 5.

12. Particule de lipoprotéine multimérique mixte comprenant une protéine hybride suivant l'une quelconque des revendications 1 à 5 et l'antigène de surface de l'hépatite B.

13. Particule suivant la revendication 12, dans laquelle le rapport de la protéine hybride à l'antigène de surface est d'environ 1:4.

14. Vaccin comprenant une quantité immunoprotectrice d'une particule ou d'une protéine suivant l'une quelconque des revendications 1 à 5 ou des revendications 11 et 12 en mélange avec un support ou diluant convenable.

15. Vaccin suivant la revendication 14, comprenant de plus une saponine comme adjuvant.

16. Vaccin suivant la revendication 14, comprenant de plus du monophosphoryl lipide A 3-désacylé.

17. Vaccin suivant la revendication 16, comprenant de plus de l'alun.

18. Vaccin suivant la revendication 16, présenté dans une émulsion d'huile dans l'eau.

19. Vaccin suivant l'une quelconque des revendications 14 à 18, utilisable dans le traitement d'un patient susceptible d'avoir des infections à plasmodium.

20. Utilisation d'un vaccin suivant l'une quelconque des revendications 14 à 18, dans la fabrication d'un médicament utilisable dans le traitement d'un patient susceptible d'avoir des infections à plasmodium.

21. Procédé pour la production d'une protéine hybride suivant l'une quelconque des revendications 1 à 5, lequel procédé comprend l'expression d'une séquence d'ADN codant la protéine dans un hôte convenable et la récupération du produit.

22. Procédé pour la production de particules suivant l'une quelconque des revendications 11 à 13, comprenant l'expression d'une séquence d'ADN codant une protéine suivant les revendications 1 à 4 et un antigène de surface d'hépatite B dans une souche de *Saccharomyces.*
